(19) Europäisches Patentamt
European Patent Office
Office européen des brevets

(11) Veröffentlichungsnummer : **0 057 360**
**B1**

(12) # EUROPÄISCHE PATENTSCHRIFT

(45) Veröffentlichungstag der Patentschrift : **26.09.84**

(51) Int. Cl.³ : **C 07 D319/06**, C 07 D493/10, C 08 G 63/62

(21) Anmeldenummer : **82100285.4**

(22) Anmeldetag : **16.01.82**

(54) **Neue cyclische Kohlensäurederivate, ein Verfahren zu ihrer Herstellung und ihre Verwendung als Copolymerisationskomponenten bei der Herstellung von Polycarbonaten.**

(30) Priorität : **30.01.81 DE 3103139**
**30.01.81 DE 3103138**
**30.01.81 DE 3103136**

(43) Veröffentlichungstag der Anmeldung : **11.08.82 Patentblatt 82/32**

(45) Bekanntmachung des Hinweises auf die Patenterteilung : **26.09.84 Patentblatt 84/39**

(84) Benannte Vertragsstaaten :
**AT DE FR GB IT NL SE**

(56) Entgegenhaltungen :
**JOURNAL OF ORGANIC CHEMISTRY, Band 27, 1962 WASHINGTON (US) S. SEARLES Jr. et al.: "Synthesis of Cyclic Sulfides from Cyclic Carbonate Esters. I. Thietanes" Seiten 2828-32**

(73) Patentinhaber : **BAYER AG**
**Konzernverwaltung RP Patentabteilung**
**D-5090 Leverkusen 1 Bayerwerk (DE)**

(72) Erfinder : **Krimm, Heinrich, Dr.**
**Heyenbaumstrasse 65**
**D-4150 Krefeld 1 (DE)**
Erfinder : **Buysch, Hans-Josef, Dr.**
**Brandenburger Strasse 28**
**D-4150 Krefeld 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 057 360 B1

Jouve, 18, rue St-Denis, 75001 Paris, France

**Beschreibung**

Aus J. Org. Chem. *27,* 2828 bis 2831 (1962) ist die Verbindung 1,3-Dioxan-5-hydroxymethyl-5-methyl-2-on bekannt. Die Herstellung dieser Verbindung wird dort nicht genau beschrieben und sie wird als instabil bezeichnet. Weiterhin wird dort beschrieben, daß man aus cyclischen Kohlensäureestern durch Umsetzung mit Kaliumthiocyanat cyclische Sulfide herstellen kann.

Die vorliegende Erfindung betrifft cyclische Kohlensäurederivate der Formel (I)

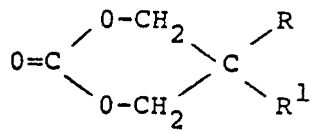

(I)

in der R entweder $-CH_2OH$ bedeutet, wobei dann $R^1$ für einen Ethylrest steht, oder

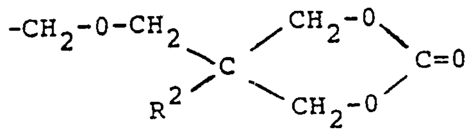

bedeutet, wobei dann $R^1$ und $R^2$ unabhängig voneinander für Methyl oder Ethyl stehen, oder

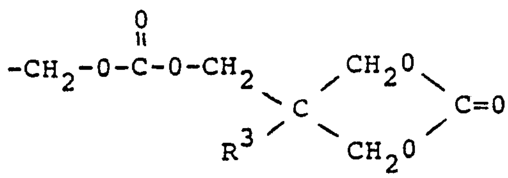

bedeutet, wobei dann $R^1$ und $R^3$ jeweils für einen Alkylrest mit 1 bis 4 C-Atomen stehen, oder in der R und $R^1$ zusammen

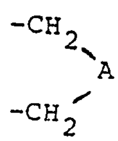

bedeuten, wobei A für die Gruppe

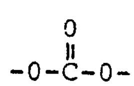

oder für Sauerstoff steht.

Die wichtigsten der neuen Kohlensäurederivate sind folgende :

a)

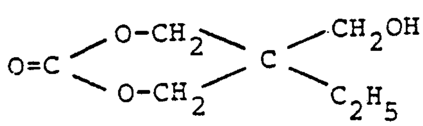

(II)

1,3-Dioxan-5-hydroxymethyl-5-ethyl-2-on oder Trimethylolpropanmonocarbonat

b)

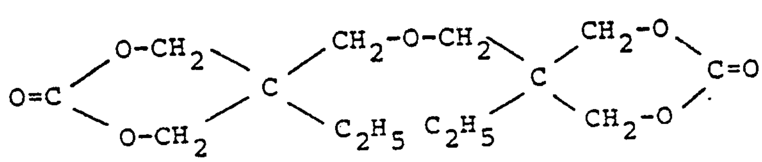

(III)

5,5'-[Oxabis(methylen)] bis [ethyl-1,3-dioxan-2-on] oder Di-trimethylolpropan-dicarbonat

c) 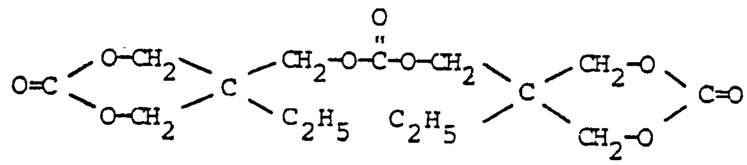 (IV)

5,5'-[Carbonylbis(oxymethylen)] bis [5-ethyl-1,3-dioxan-2-on] oder Di-(trimethylolpropancarbonat)-carbonat

d) 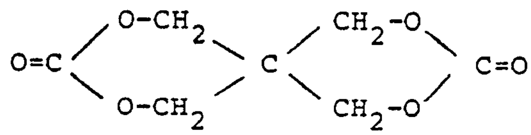 (V)

2,4,7,9-Tetraoxa-spiro [5,5] undecandion-(3,8) oder Spiro-bis-dimethylencarbonat

e) 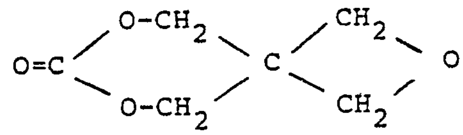 (VI)

2,4,7-Trioxa-spiro [3,5] nonanon-(3) oder Oxetan-3,3-dimethylencarbonat

Die vorliegende Erfindung betrifft weiterhin ein Verfahren zur Herstellung cyclischer Kohlensäurederivate der Formel (I)

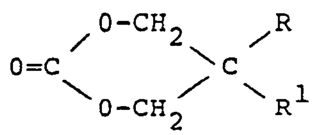 (I)

in der R entweder $-CH_2OH$ bedeutet, wobei dann $R^1$ für einen Alkylrest mit 1 bis 4 C-Atomen steht (Fall 1), oder

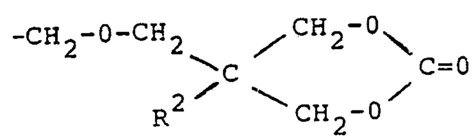

bedeutet, wobei dann $R^1$ und $R^2$ unabhängig voneinander für Methyl oder Ethyl stehen (Fall 2), oder

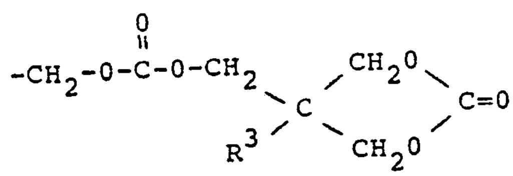

bedeutet, wobei dann $R^1$ und $R^3$ jeweils für einen Alkylrest mit 1 bis 4 C-Atomen stehen (Fall 3), oder in der R und $R^1$ zusammen

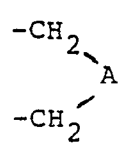

bedeuten, wobei A für die Gruppe

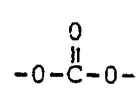

oder für Sauerstoff steht (Fall 4), das dadurch gekennzeichnet ist, daß man ein Polyol der Formel (VII)

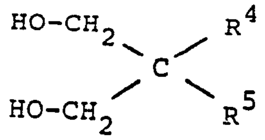

(VII)

in der $R^4$ entweder —$CH_2OH$ bedeutet, wobei dann $R^5$ für einen Alkylrest mit 1 bis 4 C-Atomen oder für —$CH_2OH$ steht oder

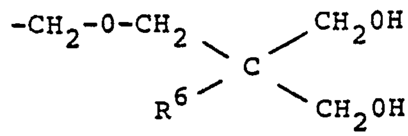

bedeutet, wobei dann $R^5$ und $R^6$ unabhängig voneinander für Methyl oder Ethyl stehen, mit einem Kohlensäurederivat aus der Gruppe, die Phosgen, Chlorkohlensäureester, Kohlensäuredialkylester und Kohlensäurediarylester umfaßt, zu einem löslichen Polycarbonat umsetzt, in denjenigen Fällen, in denen $R^4$ für —$CH_2OH$ und $R^5$ für einen Alkylrest mit 1 bis 4 C-Atomen oder $R^4$ für den Rest

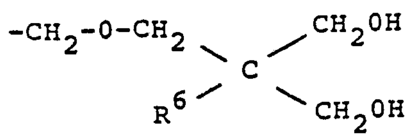

steht, den bei der Umsetzung zu einem löslichen Polycarbonat verwendeten Katalysator zwingend, in denjenigen Fällen, in denen $R^4$ für —$CH_2OH$ und $R^5$ für —$CH_2OH$ steht, diesen Katalysator gegebenenfalls entfernt, danach in den Fällen 2 und 4 das lösliche Polycarbonat in ein vernetztes, unlösliches Polycarbonat überführt, und zwar im Fall 2 bei Temperaturen von 150 bis 240 °C und Drucken von 0,001 bis 10 mbar und im Fall 4 bei Temperaturen von 100 bis 250 °C und Drucken von 1 bis 140 mbar, das dann vorliegende lösliche oder unlösliche Polycarbonat depolymerisiert, und zwar in den Fällen 1 und 3 bei Temperaturen von 150 bis 240 °C und Drucken von 0,001 bis 25 mbar, im Fall 2 bei Temperaturen von 240 bis 320 °C und Drucken von 0,001 bis 2 mbar und im Fall 4 bei Temperaturen von 200 bis 300 °C und Drucken von 0,001 bis 1 mbar und die sich dabei bildenden Kohlensäurederivate der Formel (I) abtrennt.

Bei der Herstellung von Kohlensäurederivaten der Formel (I) mit R = —$CH_2OH$ und $R^1$ = $C_1$- bis $C_4$-Alkyl verfährt man vorzugsweise so, daß man aus einem Trimethylolalkan der Formel (VIII)

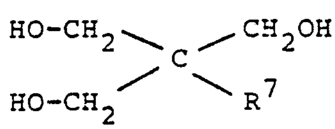

(VIII)

$R^7$ = $C_1$- bis $C_4$-Alkyl
und einer äquimolaren Menge eines Kohlensäurederivates aus der Gruppe, die Phosgen, Chlorkohlen säureester, Kohlensäuredialkylester und Kohlensäurediarylester umfaßt, unter Zusatz von Natrium- oder Kaliumhydroxid, -carbonat oder -phenolat als Katalysator ein lösliches Polycarbonat herstellt, die verwendeten Katalysatoren, deren Folgeprodukte und/oder sonstige Fremdstoffe entfernt, das so vorbehandelte lösliche Polycarbonat auf Temperaturen von 150 bis 240 °C erhitzt und die sich dabei bildenden Kohlensäurederivate der Formel (I) mit R = —$CH_2OH$ und $R^1$ = $C_1$- bis $C_4$-Alkyl abtrennt.

Vorzugsweise setzt man dabei als Trimethylolalkan Trimethylolethan oder Trimethylolpropan ein, d. h. in Formel (VIII) ist $R^7$ vorzugsweise Methyl oder Ethyl. Die Entfernung der Katalysatoren, deren Folgeprodukte und/oder sonstiger Fremdstoffe kann auf verschiedene Weise erfolgen.

Vorzugsweise löst man dazu das Polycarbonat zunächst in einem geeigneten Lösungsmittel, z. B. in Methylenchlorid, Methylenchlorid und Dioxan oder Toluol. Die so erhaltene Polycarbonatlösung kann dann beispielsweise mit Wasser, insbesondere mit destilliertem oder demineralisiertem Wasser, gewaschen werden, beispielsweise 2- bis 5-mal.

Vorzugsweise behandelt man dabei die Polycarbonatlösung vor der Wasserwäsche mit einer verdünnten Säure, z. B. mit bis zu 20 gew.-%iger wäßriger Salzsäure oder Schwefelsäure. Die Entfernung

der zur Herstellung der Polycarbonate verwendeten Katalysatoren, deren Folgeprodukte und/oder sonstiger vorhandener Fremdstoffe kann auch durchgeführt werden, indem man die Polycarbonatlösung, hierzu sind auch Lösungen in mit Wasser mischbaren Lösungsmitteln wie Aceton oder Dioxan geeignet, mit einem Ionenaustauscher behandelt. Vorzugsweise werden hierzu säureaktivierte Bleicherden und/oder sulfonierte vernetzte Polystyrole eingesetzt. Im allgemeinen ist es vorteilhaft, die zur Entfernung der bei der Herstellung der Polycarbonate verwendeten Katalysatoren, deren Folgeprodukte und/oder sonstiger Fremdstoffe benutzten Hilfsstoffe (z. B. Wasser, Lösungsmittel, Säuren und/oder Ionenaustauscher) vor der Depolymerisation möglichst vollständig zu entfernen.

Die Depolymerisation des so vorbehandelten Polycarbonats erfolgt dann durch Erhitzen. Hierzu sind Temperaturen im Bereich von 150 bis 240 °C erforderlich. Vorzugsweise arbeitet man dabei bei Temperaturen im Bereich von 160 bis 220 °C.

Die während der Depolymerisation entstehenden cyclischen Kohlensäureester der Formel (I) mit R = $-CH_2OH$ und $R^1$ = $C_1$- bis $C_4$-Alkyl werden vorzugsweise in dem Maße entfernt, wie sie sich bilden. Dies kann beispielsweise erfolgen, indem man die Depolymerisation im Vakuum durchführt. Das Vakuum wird dabei zweckmäßigerweise so gewählt, daß die gebildeten cyclischen Kohlensäureester abdestillieren und nach Kühlung in einer Vorlage aufgefangen werden können. Je nach der Flüchtigkeit der gebildeten cyclischen Kohlensäureester wird bei Drucken im Bereich 0,001 bis 25 mbar gearbeitet. Man kann die gebildeten cyclischen Kohlensäureester aber auch beispielsweise dadurch entfernen, daß man durch das Gefäß, in dem die Depolymerisation stattfindet, bei Drucken von 0,001 bis 25 mbar ein inertes Gas leitet und aus dem das Depolymerisationsgefäß verlassenden Gasstrom den gebildeten cyclischen Kohlensäureester abtrennt. Als inerte Gase kommen dafür beispielsweise Stickstoff, Kohlendioxid oder Edelgase in Frage.

Die cyclischen Kohlensäureester der Formel (I) mit R = $-CH_2OH$ und $R^1$ = $C_1$- bis $C_4$-Alkyl gehen bei dieser Depolymerisation bei konstanter Temperatur und ohne merkliche Zersetzung (Decarboxylierung) über und können nach Kühlung als kristalline Monomere in guten Ausbeuten isoliert werden. Die zu depolymerisierenden Polycarbonate neigen dazu, während der Depolymerisation zu gelieren, sie lassen sich jedoch auch in diesem Zustand ohne Schwierigkeiten nahezu vollständig depolymerisieren.

Vielfach sind die bei der Depolymerisation anfallenden cyclischen Kohlensäureester der Formel (I) mit R = $-CH_2OH$ und $R^1$ = $C_1$- bis $C_4$-Alkyl schon genügend rein für weitere Verwendungen. Sie können jedoch, falls dies erwünscht ist, weiter gereinigt werden. Diese Reinigung kann beispielsweise durch Umkristallisation aus einem geeigneten Lösungsmittel (z. B. Essigsäureethylester, Toluol, Cyclohexan, Diethylether oder Tetrachlorkohlenstoff) oder durch eine Destillation unter vermindertem Druck erfolgen.

Bei der Herstellung der neuen Kohlensäurederivate der Formel (I) mit R und $R^1$ zusammen gleich

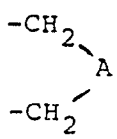

wobei A für die Gruppe

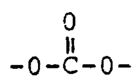

oder für Sauerstoff steht, verfährt man vorzugsweise so, daß man

a) Pentaerythrit mit einem Kohlensäurederivat aus der Gruppe, die Phosgen, Chlorkohlensäureester, Kohlensäuredialkylester und Kohlensäurediarylester umfaßt, gegebenenfalls in Gegenwart basisch wirkender Verbindungen, zu einem löslichen Vorprodukt umsetzt,

b) danach gegebenenfalls die basisch wirkenden Verbindungen entfernt,

c) aus dem löslichen Vorprodukt durch Erhitzen auf 100 bis 250 °C bei Drucken im Bereich von 1 bis 140 mbar ein vernetztes Polycarbonat herstellt,

d) das vernetzte Polycarbonat, gegebenenfalls in Gegenwart von Katalysatoren, bei 200 bis 300 °C und Drucken im Bereich von 0,001 bis 1 mbar depolymerisiert und

e) die im Schritt d) absublimierenden oder abdestillierenden bicyclischen Kohlensäurederivate der Formel (I) mit R und $R^1$ zusammen gleich

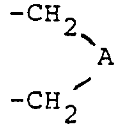

wobei A für die Gruppe

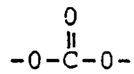

oder Sauerstoff steht, auffängt und gegebenenfalls trennt.

Die im Schritt a) dieses Verfahrens durchzuführende Umsetzung von Pentaerythrit kann im Prinzip mit Phosgen, Chlorkohlensäureestern oder Kohlensäuredialkyl- oder -diarylestern durchgeführt werden. Aus ökologischen Gründen ist die Umsetzung mit Kohlensäuredialkyl- oder -diarylestern bevorzugt, weshalb im folgenden im wesentlichen auf diese Arbeitsweise eingegangen wird. Dem Fachmann bereitet es keine Schwierigkeiten, entsprechende Umsetzungen mit Phosgen oder Chlorkohlensäureestern durchzuführen.

Wesentlich bei Schritt a) ist, daß die Umsetzung zu einem löslichen Vorprodukt und nicht zu unlöslichen, stark vernetzten Polymeren führt. Dies kann beispielsweise erreicht werden, indem man einen großen Überschuß an Kohlensäuredialkyl- oder -diarylester einsetzt, beispielsweise pro Mol Pentaerythrit 4 bis 10 Mol Kohlensäureester.

Die Umsetzung mit Kohlensäureestern wird vorzugsweise in Gegenwart von Umesterungskatalysatoren durchgeführt. Als Beispiele für solche Umesterungskatalysatoren seien genannt : Oxide, Hydroxide, Alkoholate, Carboxylate und Carbonate von Alkalimetallen, insbesondere Natrium und Kalium, sowie von Aluminium, Thallium oder Blei, die beispielsweise in Mengen von 0,1 bis 2 g pro Mol Pentaerythrit eingesetzt werden können.

Die Umsetzung zwischen Kohlensäureester und Pentaerythrit kann beispielsweise so durchgeführt werden, daß man die Komponenten mischt, einen oder mehrere Umesterungskatalysatoren zufügt und die Mischung erhitzt, beispielsweise auf Temperaturen im Bereich von 120 bis 140 °C und den entstehenden Alkohol, gegebenenfalls unter vermindertem Druck und gegebenenfalls über eine Kolonne, abdestilliert. Beim Einsatz von Diethylcarbonat als Kohlensäureester kann man beispielsweise das Reaktionsgemisch am Rückfluß bei einer Temperatur von 120 bis 130 °C sieden lassen und über eine Kolonne bei 78 bis 80 °C den entstehenden Ethylalkohol abtrennen.

Wird anstelle von Kohlensäureester mit Phosgen oder Chlorkohlensäureester, beispielsweise Chlorkohlensäurephenylester, umgesetzt, so arbeitet man vorzugsweise in Gegenwart eines Lösungsmittels, beispielsweise Essigsäureethylester, und eines säurebindenden Mittels, beispielsweise Dimethylanilin, bei Temperaturen von beispielsweise 20 bis 80 °C. Nach Beendigung der Umsetzung sind in diesem Fall das Lösungsmittel und das aus dem säurebindenden Mittel entstandene Salz zu entfernen, beispielsweise durch Waschen mit Wasser und Abziehen des Lösungsmittels unter vermindertem Druck.

Wenn im Schritt a) basisch wirkende Verbindungen eingesetzt worden sind, beispielsweise als Umesterungskatalysatoren, so sind diese im Schritt b) zu entfernen, wenn man das Spiro-bis-dimethylencarbonat (s. Formel (V)) herstellen will. Wenn man Oxetan-3,3-dimethylencarbonat (s. Formel (VI)) herstellen will, können solche basisch wirkenden Verbindungen in dem im Schritt a) hergestellten Vorprodukt verbleiben oder entfernt werden.

Die Entfernung der basisch wirkenden Verbindungen kann auf verschiedene Weise vorgenommen werden. Vorzugsweise verfährt man dabei so, wie bei der Herstellung von Kohlensäurederivaten der Formel (I) mit $R = -CH_2OH$ und $R^1 = C_1$- bis $C_4$-Alkyl beschrieben.

Im allgemeinen ist es vorteilhaft, die zur Entfernung der basisch wirkenden Verbindungen benutzten Hilfsstoffe (z. B. Wasser, Säuren, Lösungsmittel und/oder Ionenaustauscher) vor der Durchführung des Schrittes c) möglichst vollständig zu entfernen.

Im Schritt c) wird dann aus dem löslichen, gegebenenfalls von basisch wirkenden Verbindungen befreiten, Vorprodukt durch Erhitzen auf 100 bis 250 °C bei Drucken im Bereich von 1 bis 140 mbar ein vernetztes Polycarbonat hergestellt. Bevorzugt arbeitet man dabei bei Temperaturen im Bereich 140 bis 210 °C und Drucken im Bereich 5 bis 50 mbar. Die Druck- und Temperaturbedingungen sollten dabei im allgemeinen innerhalb der angegebenen Grenzen so eingestellt werden, daß keine Pentaerythritderivate abdestillieren oder absublimieren, sondern nur überschüssiges Ausgangsmaterial, z. B. Diethylcarbonat, und/oder bei der Bildung des vernetzten Polycarbonats entstehende Produkte, z. B. Diethylcarbonat oder, im Falle des Einsatzes von Chlorkohlensäurephenylester im Schritt a), Phenol. Nach Durchführung des Schrittes c) verbleibt das gebildete vernetzte Polycarbonat als Rückstand.

Unter Umständen, beispielsweise wenn in Schritt a) Pentaerythrit mit einem Dialkylcarbonat umgesetzt und im Schritt b) basisch wirkende Verbindungen abgetrennt worden sind, während der Durchführung des Schrittes c) keine Katalysatoren zugegen sind und bei sehr geringen Drucken, beispielsweise unter 0,1 mbar gearbeitet wird, ist es möglich, durch schnelle Destillation nichtbicyclische Pentaerythrit-Derivate wie Pentaerythrit-monocarbonat-bis-alkyl-carbonate (s. Formel (IX)) und Pentaerythrit-tetrakis-alkylcarbonate (s. Formel (X)) abzutrennen.

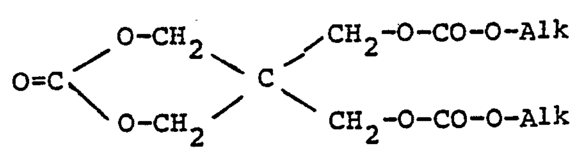

Alk = Alkyl (IX)

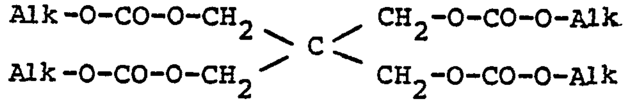

Alk = Alkyl (X)

Aus den Verbindungen, die den Formeln (IX) und (X) entsprechen, können die Verbindungen der Formel (I) mit R und $R^1$ zusammen gleich

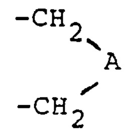

wobei A für die Gruppe

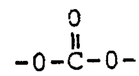

oder Sauerstoff steht, erhalten werden, indem sie den Verfahrensschritten c), d) und e) unterworfen werden.

Im Schritt d) wird das vernetzte Polycarbonat bei Temperaturen im Bereich von 200 bis 300 °C, bevorzugt 220 bis 280 °C, und bei Drucken im Bereich von 0,001 bis 1 mbar, bevorzugt 0,01 bis 0,1 mbar, depolymerisiert.

Dabei destillieren oder sublimieren die Verbindungen entsprechend der Formel (I) mit R und $R^1$ zusammen gleich

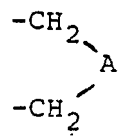

wobei A für die Gruppe

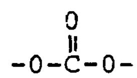

oder Sauerstoff steht, ab. Innerhalb der angegebenen Temperatur- und Druckgrenzen werden vorzugsweise solche Bedingungen gewählt, unter denen die Verbindungen entsprechend der Formel (I) mit R und $R^1$ zusammen gleich

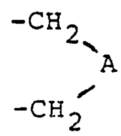

wobei A für die Gruppe

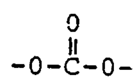

oder Sauerstoff steht, absublimieren.

Von der Gegenwart bzw. Abwesenheit von Katalysatoren im Schritt d) des erfindungsgemäßen Verfahrens hängt es ab, ob Spiro-bis-dimethylencarbonat (s. Formel (V)) und/oder Oxetan-3,3-dimethylencarbonat (s. Formel (VI)) als Reaktionsprodukt erhalten wird. Bei Abwesenheit von Katalysatoren oder in Gegenwart schwach wirksamer Katalysatoren wird praktisch ausschließlich Spiro-bis-dimethylencarbonat erhalten, bei Anwesenheit stärker wirksamer Katalysatoren wird Kohlendioxid abgespalten und praktisch ausschließlich Oxetan-3,3-dimethylencarbonat erhalten. Schwach wirksame Katalysatoren sind z. B. organische Verbindungen des 2- oder 4-wertigen Zinns oder des 4-wertigen Titans, wie Zinn-(II)-di-octoat, Zinn-(II)-dilaurat, Dibutylzinndilaurat, Titantetrabutylat, Titantetraisooctylat oder Titantetradodecylat. Schwach wirksame Katalysatoren können in Mengen von beispielsweise 0,01 bis 1 Gew.-%, vorzugsweise 0,01 bis 0,1 Gew.-%, bezogen auf die Menge des Polycarbonats, eingesetzt werden. Der Zusatz von schwach wirksamen Katalysatoren hat gegenüber der Arbeitsweise in Abwesenheit von Katalysatoren den Vorteil, daß die zur Bildung des Spiro-bis-dimethylencarbonats führende Umesterung beschleunigt wird, wodurch im Schritt d) die Reaktionszeit verkürzt und/oder die Reaktionstemperatur erniedrigt werden kann.

Stärker wirksame Katalysatoren sind beispielsweise solche, die im Schritt a) bei der Umsetzung von Pentaerythrit mit Kohlensäureestern eingesetzt werden können, also beispielsweise Oxide, Hydroxide, Alkoholate, Carboxylate und Carbonate von Alkalimetallen, insbesondere Natrium und Kalium, sowie von Aluminium, Thallium oder Blei. Stärker wirksame Katalysatoren können in Mengen von beispielsweise 0,001 bis 0,5 Gew.-%, vorzugsweise 0,01 bis 0,1 Gew.-%, bezogen auf die Menge des Polycarbonats, eingesetzt werden.

Falls im Schritt d) in Gegenwart von Katalysatoren gearbeitet werden soll, kann die Zugabe der Katalysatoren zu verschiedenen Zeitpunkten innerhalb des Verfahrens erfolgen. Sind bereits bei der Umsetzung von Pentaerythrit mit Kohlensäureestern im Schritt a) stärker wirksame Katalysatoren eingesetzt worden, so können diese während des gesamten Verfahrens zugegen bleiben. Schwach wirksame und stärker wirksame Katalysatoren können beispielsweise nach dem Schritt b) und/oder nach dem Schritt c) zugegeben werden. Wenn im Schritt d) in Abwesenheit von Katalysatoren gearbeitet werden soll, kann der Schritt b) des Verfahrens nicht entfallen.

Die im Schritt e) aufgefangenen Kohlensäurederivate der Formel (I) mit R und $R^1$ zusammen gleich

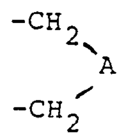

wobei A für die Gruppe

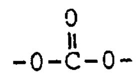

oder Sauerstoff steht, sind im allgemeinen noch nicht völlig rein. Falls gewünscht, können sie, beispielsweise durch Umkristallisieren oder Sublimation, gereinigt werden. Für die Umkristallisation des Spiro-bis-dimethylencarbonats sind beispielsweise Dioxan, Diethylcarbonat oder Essigsäureethylester geeignet, für die Umkristallisation von Oxetan-3,3-dimethylencarbonat beispielsweise Essigsäureethylester.

Wie vorstehend ausgeführt kann man den Schritt d) des Verfahrens so durchführen, daß praktisch nur jeweils eine der beiden Verbindungen entsprechend den Formeln (V) oder (VI) erhalten wird. Wenn die bei der Beschreibung des Schrittes d) angegebenen Parameter und Katalysatoren jedoch nicht optimal eingehalten bzw. gewählt werden, können im Schritt e) auch Gemische der Verbindungen entsprechend den Formeln (V) und (VI) aufgefangen werden. Solche Gemische können auf einfache Weise aufgetrennt werden, da Oxetan-3,3-dimethylencarbonat (s. Formel (VI)) in Methylenchlorid gut löslich, Spiro-bis-dimethylcarbonat (s. Formel (V)) in Methylenchlorid jedoch praktisch unlöslich ist.

Bei der Herstellung der neuen Kohlensäurederivate der Formel (I) mit R =

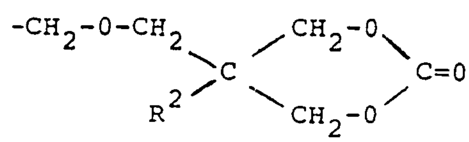

und $R^1$ und $R^2$ unabhängig voneinander
= Methyl oder Ethyl verfährt man vorzugsweise so, daß man Di-trimethylolalkane der Formel (XII)

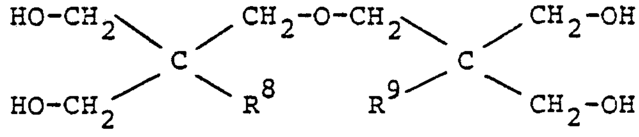

(XII)

in der
$R^8$ und $R^9$ gleich oder verschieden sein können und für Methyl oder Ethyl stehen,

a) mit einem Kohlensäurederivat aus der Gruppe, die Phosgen, Chlorkohlensäureester, Kohlensäuredialkylester und Kohlensäurediarylester umfaßt, zu einem löslichen Vorprodukt umsetzt,

b) falls im Schritt a) in Gegenwart basisch wirkender Verbindung gearbeitet wurde, diese basisch wirkenden Verbindungen entfernt,

c) in Abwesenheit von Katalysatoren oder in Anwesenheit schwach wirksamer Umesterungskatalysatoren aus der Gruppe der organischen Verbindungen des 2- und 4-wertigen zinns und der 4-wertigen

Titans durch Erhitzen auf Temperaturen im Bereich von 150 bis 240 °C und bei Drucken im Bereich von 0,001 bis 10 mbar aus dem löslichen Vorprodukt ein vernetztes Polycarbonat herstellt und

d) das vernetzte Polycarbonat bei Temperaturen von 240 bis 320 °C und Drucken im Bereich von 0,001 bis 2 mbar depolymerisiert und das übergehende bicyclische Ethercarbonat der Formel (I) mit R =

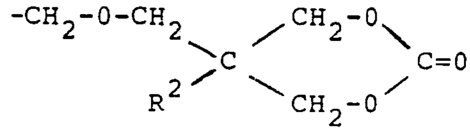

und $R^1$ und $R^2$ unabhängig voneinander = Methyl oder Ethyl, in einer gekühlten Vorlage auffängt.

Die in das erfindungsgemäße Verfahren einzusetzenden Di-trimethylolalkane, beispielsweise Di-trimethylolethan (Formel XII, $R^8$ und $R^9$ = Methyl) oder Di-trimethylolpropan (Formel XII, $R^8$ und $R^9$ = Ethyl) sind bei der großtechnischen Herstellung von Trimethylolalkanen anfallende Nebenprodukte.

Die im Schritt a) des erfindungsgemäßen Verfahrens durchzuführende Umsetzung mit einem der angegebenen Kohlensäurederivate kann im Prinzip mit Phosgen, Chlorkohlensäureestern oder Kohlensäuredialkyl- oder -diarylestern durchgeführt werden. Aus ökologischen Gründen ist die Umsetzung mit Kohlensäuredialkyl- oder -diarylestern bevorzugt, weshalb im folgenden im wesentlichen auf diese Arbeitsweise eingegangen wird. Dem Fachmann bereitet es keine Schwierigkeiten, entsprechende Umsetzungen mit Phosgen oder Chlorkohlensäureestern durchzuführen.

Wesentlich bei Schritt a) ist, daß die Umsetzung zu einem löslichen Produkt und nicht zu einem unlöslichen stark vernetzten Polymeren führt. Dies kann beispielsweise erreicht werden, indem man einen großen Überschuß an Kohlensäuredialkyl- oder -diarylester einsetzt, beispielsweise pro Mol einer Verbindung der Formel (XII) 4 bis 10 Mol Kohlensäureester.

Die Umsetzung mit Kohlensäureestern wird vorzugsweise in Gegenwart von Umesterungskatalysatoren durchgeführt, um die Reaktionsgeschwindigkeit zu erhöhen. Als Beispiele für solche Umesterungskatalysatoren seien genannt : Oxide, Hydroxide, Alkoholate, Carboxylate und Carbonate von Alkalimetallen, insbesondere von Natrium und/oder Kalium, sowie von Aluminium, Thallium oder Blei, die beispielsweise in Mengen von 0,2 bis 4 g pro Mol Verbindung der Formel (XII) eingesetzt werden können.

Die Umsetzung zwischen Kohlensäureestern und Verbindungen der Formel (XII) kann beispielsweise so durchgeführt werden, daß man die Komponenten mischt, einen oder mehrere Umesterungskatalysatoren zufügt und die Mischung erhitzt, beispielsweise auf Temperaturen im Bereich von 120 bis 140 °C und den entstehenden Alkohol, gegebenenfalls unter vermindertem Druck und gegebenenfalls über eine Kolonne, abdestilliert. Beim Einsatz von Diethylcarbonat als Kohlensäureester kann man beispielsweise das Reaktionsgemisch am Rückfluß bei einer Temperatur von bis zu 130 °C sieden lassen und über eine Kolonne bei 78 bis 80 °C den entstehenden Ethylalkohol abtrennen.

Wird anstelle von Kohlensäureester mit Phosgen oder Chlorkohlensäureester, beispielsweise Chlorkohlensäurephenylester, umgesetzt, arbeitet man vorzugsweise in Gegenwart eines Lösungsmittels, beispielsweise Essigsäureethylester, und eines säurebindenden Mittels, beispielsweise Dimethylanilin, bei Temperaturen von beispielsweise 20 bis 80 °C. Nach Beendigung der Umsetzung sind in diesem Fall das Lösungsmittel und das aus dem säurebindenden Mittel entstandene Salz zu entfernen, beispielsweise durch Waschen mit Wasser und Abziehen des Lösungsmittels unter vermindertem Druck.

Wenn im Schritt a) basisch wirkende Verbindungen eingesetzt worden sind, beispielsweise als Umesterungskatalysatoren oder als säurebindende Mittel, so sind diese im Schritt b) zu entfernen. Die Entfernung der basisch wirkenden Verbindungen kann auf verschiedene Weise vorgenommen werden. Vorzugsweise verfährt man auch hier so wie bei der Herstellung von Kohlensäurederivaten der Formel (I) mit R = $-CH_2OH$ und $R^1$ = $C_1$- bis $C_4$-Alkyl beschrieben.

Im allgemeinen ist es vorteilhaft, die zur Entfernung der basisch wirkenden Verbindungen benutzten Hilfsstoffe (z. B. Wasser, Säuren, Lösungsmittel und/oder Ionenaustauscher) vor der Durchführung des Schrittes c) möglichst vollständig zu entfernen. Ionenaustauscher können beispielsweise durch Filtration oder durch Verwendung einer geeigneten Säule, Lösungsmittel beispielsweise durch Abziehen unter vermindertem Druck, entfernt werden.

Im Schritt c) wird dann aus dem löslichen, von basisch wirkenden Verbindungen befreiten Vorprodukt durch Erhitzen auf 150 bis 240 °C bei Drucken im Bereich von 0,001 bis 10 mbar ein vernetztes Polycarbonat hergestellt. Dabei können noch vorhandene oder sich bildende leichter flüchtige Substanzen als die Verbindungen der Formel (I) mit R =

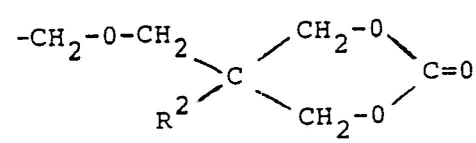

und $R^1$ und $R^2$ unabhängig voneinander = Methyl oder Ethyl, abdestillieren, z. B. Diethylcarbonat. Nach Durchführung des Schrittes c) verbleibt das gebildete, vernetzte Polycarbonat als Rückstand.

Der Schritt c) des erfindungsgemäßen Verfahrens wird in Abwesenheit von Katalysatoren oder in Gegenwart schwach wirksamer Umesterungskatalysatoren aus der Gruppe der organischen Verbindungen des 2- und 4-wertigen Zinns und des 4-wertigen Titans, wie Zinn-(II)-dioctoat, Zinn-(II)-dilaurat, Dibutylzinndilaurat, Zinn-(II)-diacetat, Titantetrabutylat, Titantetraisooctylat oder Titantetradodecylat durchgeführt. Solche schwach wirksamen Umesterungskatalysatoren können in Mengen von beispielsweise 0,001 bis 0,5 Gew.-%, vorzugsweise 0,01 bis 0,1 Gew.-%, bezogen auf die Menge des Polycarbonats eingesetzt werden. Der Zusatz von schwach wirksamen Umesterungskatalysatoren hat gegenüber der Arbeitsweise in Abwesenheit von Katalysatoren den Vorteil, daß höhere Ausbeuten an Dicarbonaten erzielt werden können.

Im Schritt d) wird das vernetzte Polycarbonat bei Temperaturen im Bereich von 240 bis 320 °C, bevorzugt 250 bis 280 °C, und bei Drucken im Bereich von 0,001 bis 2 mbar, bevorzugt 0,05 bis 2 mbar, depolymerisiert. Dabei destillieren oder sublimieren die Verbindungen entsprechend der Formel (I) mit R =

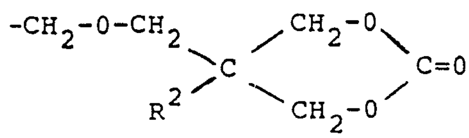

und $R^1$ und $R^2$ unabhängig voneinander = Methyl oder Ethyl ab. Innerhalb der angegebenen Temperatur- und Druckgrenzen werden vorzugsweise solche Bedingungen gewählt, unter denen die Verbindungen entsprechend der Formel (I) mit R =

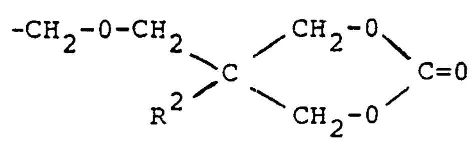

und $R^1$ und $R^2$ unabhängig voneinander = Methyl oder Ethyl abdestillieren. Diese Verbindungen können auch zum Übergehen gebracht oder deren Übergehen erleichtert werden, indem man bei den angegebenen Bedingungen ein inertes Gas, wie Stickstoff, Kohlendioxid oder ein Edelgas, durch das Gefäß leitet, in dem die Depolymerisation stattfindet.

Die im Schritt d) übergehenden Verbindungen können in einer gekühlten Vorlage aufgefangen werden. Diese Verbindungen fallen dann im allgemeinen als kristallisierende Masse an. Falls gewünscht können diese Verbindungen noch gereinigt werden, beispielsweise durch Umkristallisation oder nochmalige Destillation. Ein geeignetes Lösungsmittel für die Umkristallisation ist beispielsweise Toluol.

Die Herstellung der neuen Kohlensäurederivate der Formel (I) mit R =

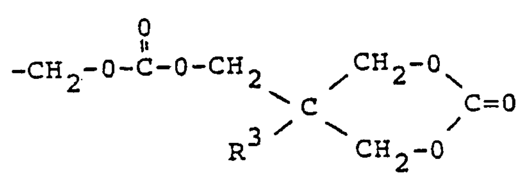

und $R^1$ und $R^3$ jeweils = $C_1$- bis $C_4$-Alkyl erfolgt vorzugsweise so, wie vorstehend für die Herstellung der neuen Kohlensäurederivate der Formel (I) mit R = —$CH_2OH$ und $R^1$ = $C_1$- bis $C_4$-Alkyl beschrieben und anschließender Umsetzung der erhaltenen Produkte mit Phosgen in Gegenwart basischer Verbindungen, z. B. Pyridin.

Bei der erfindungsgemäßen Umsetzung eines Polyols der Formel (VII) mit einem Kohlensäurederivat aus der Gruppe, die Phosgen, Chlorkohlensäureester, Kohlensäuredialkylester und Kohlensäurediarylester umfaßt, wird vorzugsweise ein Carbonat der Formel (XIII)

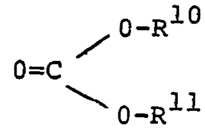

(XIII)

worin $R^{10}$ und $R^{11}$ gleich oder verschieden sein können und für $C_1$- bis $C_4$-Alkyl oder Phenyl sthen oder worin $R^{10}$ und $R^{11}$ gemeinsam für die Gruppe $CH_2$—$CH_2$ oder $CH_2$—CH—$CH_3$ steht, eingesetzt. Aus ökologischen Gründen wird besonders bevorzugt Diethylcarbonat eingesetzt (Formel (XIII), $R^{10}$ und $R^{11}$ gleich Ethyl).

Es ist ausgesprochen überraschend, daß es erfindungsgemäß gelingt, die cyclischen Kohlensäureester der Formel (I) herzustellen und zu isolieren, denn gemäß J.A.C.S. *79* 3455 (1957) zersetzt sich das Carbonat des Trimethylolpropans unter Decarboxylierung und Bildung von Hydroxymethyloxetanen schon bei 180 bis 200 °C.

Die vorliegende Erfindung betrifft weiterhin die Verwendung von cyclischen Kohlensäurederivaten der Formel (I)

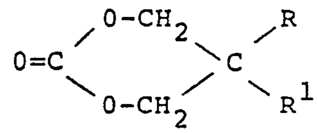

(I)

in der R entweder $—CH_2OH$ bedeutet, wobei dann $R^1$ für einen Alkylrest mit 1 bis 4 C-Atomen steht, oder

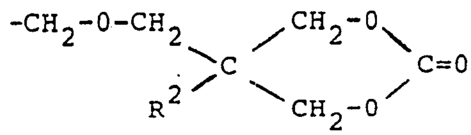

bedeutet, wobei dann $R^1$ und $R^2$ unabhängig voneinander für Methyl oder Ethyl stehen, oder

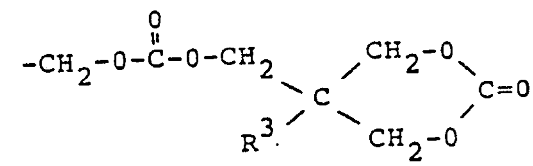

bedeutet, wobei dann $R^1$ und $R^3$ jeweils für einen Alkylrest mit 1 bis 4 C-Atomen stehen, oder in der R und $R^1$ zusammen

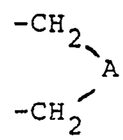

bedeuten, wobei A für die Gruppe

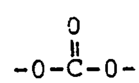

oder für Sauerstoff steht, als Copolymerisationskomponenten zur Herstellung von Polycarbonaten.

Beispielsweise erhält man aus den Verbindungen der Formel (I), wenn man sie zusammen mit anderen organischen Carbonaten, z. B. Neopentylglykolcarbonat, mit basischen Katalysatoren bei erhöhter Temperatur polymerisiert, ein unlösliches, unschmelzbares, transparentes Polymerisat. Diese Umsetzung kann in an sich bekannter Weise erfolgen. So sind erstmals heißhärtende Polycarbonate (Duromere) durch Polymerisation herstellbar, also ohne die Abspaltung von Blasen bildenden, die Umwelt belastenden Stoffen. Im Gegensatz zu den bekannten Duromeren auf der Basis von ungesättigten Polyestern und Styrol weisen die erfindungsgemäß zugänglichen Duromere eine überraschend hohe Kerbschlagzähigkeit auf, so daß sie im allgemeinen nicht mit Glasfasern verstärkt werden müssen.

## Beispiele

### Beispiel 1

268 g (2 Mol) Trimethylolpropan, 236 g (2 Mol) Diethylcarbonat und 1 g Kaliumcarbonat wurden unter Rühren an einer 1 m-Füllkörperkolonne zum Rückfluß erhitzt, während über Kopf 78 °C Ethanol abdestilliert wurde, bis die Innentemperatur auf 130 °C gestiegen und 86 g Ethanol abgetrennt waren. Durch allmähliche Verminderung des Druckes auf 30 mbar wurde die restliche Menge des Ethanols abdestilliert, insgesamt 164 g = 4 Mol. Das Reakionsprodukt, ein bei Raumtemperatur kaum mehr fließendes Harz, wurde in 700 ml Methylenchlorid/Dioxan (1 : 1) gelöst und über einen Ionenaustauscher,

einem Perlpolymerisat aus sulfoniertem, vernetztem Polystyrol, laufen gelassen. Das Lösungsmittel wurde unter vermindertem Druck entfernt und 312 g Rückstand erhalten. Durch einen auf 155 °C beheitzten Tropftrichter wurde das Rohprodukt aus einem auf 220 °C vorgeheizten Rührkolben im Laufe von 100 Minuten bei 170 °C unter einem Druck von 0,02 mbar destilliert. Dazu wurde eine mit Methanol/Trockeneis und eine mit flüssigem Stickstoff gekühlte Kältefalle verwendet. Es wurden 292 g (91 % der Theorie) farbloses Destillat erhalten, das bei Raumtemperatur kristallisierte. Der Schmelzpunkt betrug 31 bis 32 °C. Nach der Umkristallisation aus Diethylether betrug der Schmelzpunkt 40 bis 41 °C.

Elementaranalyse : $C_7H_{12}O_4$ (160,1)

C ber. : 52,51 gef. : 52,68
H ber. : 7,56 gef. : 7,41
O ber. : 39,98 gef. : 39,67

Es handelte sich um das Trimethylolpropanmonocarbonat (s. Formel II), das als Phenylurethan charakterisiert wurde, Schmelzpunkt 112 °C (aus Toluol).

Beispiel 2

In eine Lösung von 32 g (0,2 Mol) Trimethylolpropanmonocarbonat, das gemäß Beispiel 1 erhalten wurde, in 50 g Pyridin wurde bei Raumtemperatur 10,5 g (0,106 Mol) Phosgen eingeleitet und 1 Stunde bei 60 °C gehalten. Anschließend wurde mit Methylenchlorid verdünnt, das überschüssige Pyridin durch Ausschütteln mit 2 n Salzsäure entfernt, über Natriumsulfat getrocknet und das Lösungsmittel unter vermindertem Druck entfernt. Es wurden 30,2 g Rückstand erhalten, Schmelzpunkt 248 bis 254 °C (aus Essigsäureethylester umkristallisiert). Bei der Verbindung handelt es sich um Di-(trimethylol-propancarbonat)-carbonat der folgenden Formel (IV)

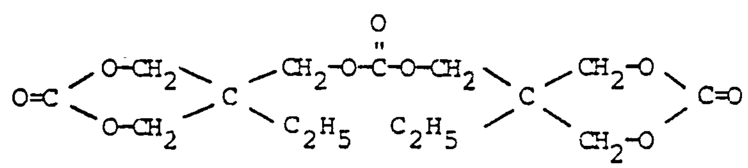

(IV)

Elementaranalyse : $C_{15}H_{22}O_9$ (346,3)

C ber. : 52,02 gef. : 52,31
H ber. : 6,41 gef. : 6,28
O ber. : 41,58 gef. : 41,30

Beispiel 3

9 g Neopentylglykolcarbonat, 1 g Di-(trimethylolpropancarbonat)-carbonat, das gemäß Beispiel 2 erhalten wurde, und 0,1 g gemahlendes Kaliumcarbonat wurden auf 130 °C erhitzt. Unter Temperaturerhöhung trat Polymerisation zu einem unschmelzbaren, in Lösungsmitteln unlöslichen opaken Reaktionsprodukt von großer Zähigkeit, Elastizität und Festigkeit ein.

Beispiel 4

240 g (2 Mol) Trimethylolethan, 260 g (2,2 Mol) Diethylcarbonat und 1 g Natriummethylat wurden unter Rühren an einer 1 n Füllkörperkolonne in der gleichen Weise wie in Beispiel 1 umgesetzt. Nach 2 Stunden 45 Minuten waren 185 g Ethanol abgetrennt. Der Rückstand, ein zähes Harz, wurde in 600 ml Methylenchlorid gelöst und über einen Ionenaustauscher, ein sulfoniertes, vernetztes Polystyrol, laufen gelassen. Das Lösungsmittel wurde unter vermindertem Druck entfernt und 286 g Rückstand erhalten. 40 g dieses Rückstandes wurden aus einem Claisen-Kolben unter Rühren bei einer Innentemperatur von 210 °C und einer Übergangstemperatur von 160 bis 180 °C destilliert. Es wurden 32 g eines kristallisierten Destillates vom Schmelzpunkt 93 bis 94 °C erhalten (aus Ether umkristallisiert). Es handelte sich dabei um Trimethylolethanmonocarbonat (s. Formel II).

Elementaranalyse : $C_6H_{10}O_4$ (146,1)

C ber. : 49,32 gef. : 49,28
H ber. : 6,90 gef. : 6,98
O ber. : 43,81 gef. : 43,62

Beispiel 5

136 g (1 Mol) Pentaerythrit, 708 g (6 Mol) Diethylcarbonat, 100 mg Thalliumcarbonat und 200 mg Bleinaphthenat wurden an einer 90 cm Füllkörperkolonne zum Rückfluß (126-128 °C) erhitzt, während am Kolonnenkopf bei 78 bis 80 °C Ethylalkolol abgenommen wurde. Nach 14 Stunden Reaktionszeit war

die berechnete Menge Ethylalkohol (184 g = 4 Mol) abgetrennt. Vom Rückstand, einer leicht trüben farblosen Flüssigkeit (628 g), wurden 70 g in einem Weithalskolben, der Teil einer Sublimationsapparatur war, unter einem Druck von 20 mbar im Laufe von 3 Stunden auf 200 °C erhitzt, um das überschüssige Diethylcarbonat abzutrennen (48 g). Das zurückbleibende zähe Harz (25 g) wurde sodann in einer Sublimationstemperatur bei 200 bis 230 °C unter einem Druck von 0,03 bis 0,05 mbar gehalten. Nach jeweils 1,5 Stunden wurde das Sublimat abgeschabt. Im Zeitraum von 10 Stunden wurden 11 g Sublimat vom Schmelzpunkt 110 bis 122 °C erhalten. Nach Umkristallisation aus Essigsäureethylester wurden 8 g farblose Kristalle vom Schmelzpunkt 135 bis 136 °C erhalten. Es handelte sich dabei um Oxetan-3,3-dimethylencarbonat der Formel (VI)

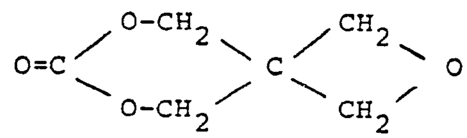

(VI)

Elementaranalyse : $C_6H_8O_4$ (144,1)
C ber. : 50,00 gef. : 49,78
H ber. : 5,99 gef. : 5,49
O ber. : 44,40 gef. : 44,63

Das $^1$H-NMR-Spektrum zeigte nur ein Signal bei 4,65 ppm, gemessen mit TMS (δ = 0 ppm) als Standard.

Die $^{13}$C-NMR-Analyse wies vier verschiedene Arten von C-Atomen aus.

Im IR-Spektrum ist die CO-Bande bei 1 740 $cm^{-1}$ und die Bande für den Oxetanring bei 980 $cm^{-1}$ charakteristisch.

Die Substanz reagiert mit etherischer Salzsäure unter Erwärmen.

## Beispiel 6

340 g (2,5 Mol) Pentaerythrit, 1 770 g (15 Mol) Diethylcarbonat und 2,5 g Thalliumcarbonat wurden in einer 1 m-Füllkörperkolonne zum Rückfluß erhitzt, während am Kolonnenkopf bei 78 bis 80 °C Ethanol abgenommen wurde. Nach 4 Stunden waren 475 g Destillat abgetrennt. Das Reaktionsprodukt wurde mit Methylenchlorid aufgenommen und zur Entfernung des Katalysators mit 10 ml 2 n Salzsäure und 2-mal mit je 200 ml Wasser ausgeschüttelt. Nach Trocknen über Natriumsulfat wurde die Lösung eingeengt und unter vermindertem Druck bei 150 °C/14 mbar vom überschüssigen Diethylcarbonat befreit. Der Rückstand betrug 819 g. 150 g davon wurden in einem Birnenkolben mit 50 mg Zinndilaurat versetzt und in einem Rotationsverdampfer unter einem Druck von 1 mbar im Laufe von 1 Stunde von 210 auf 240 °C Badtemperatur erhitzt, bis ein vernetztes Polykondensat gebildet war. Aus dem Birnenkolben, der Teil einer Sublimationsapparatur war, wurden im Laufe von 12 Stunden bei 220 bis 250 °C und 0,03 mbar 42 g einer kristallinen Substanz sublimiert, bei der es sich um Spiro-bis-dimethylencarbonat der Formel (V)

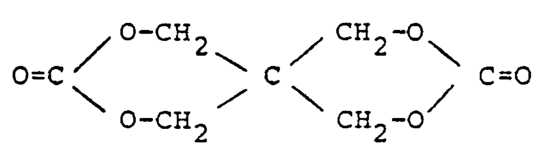

(V)

handelte. Der Schmelzpunkt dieser Substanz betrug 222 bis 228 °C (Gasentwicklung). Die Substanz erwies sich im Methylenchlorid als praktisch unlöslich und kann aus Dioxan oder Diethylcarbonat umkristallisiert werden.

Elementaranalyse : $C_7H_8O_6$ (118,1)
C ber. : 44,69 gef. : 44,31
H ber. : 4,28 gef. : 4,38
O ber. : 51,03 gef. : 50,91

Das $^1$H-NMR-Spektrum zeigte nur ein einziges Signal bei 4,65 ppm, das IR-Spektrum eine CO-Bande bei 1 741 $cm^{-1}$.

Die für ein Oxetan typische Bande bei 980 $cm^{-1}$ fehlt. Mit etherischer Salzsäure erfolgte keine Erwärmung.

## Beispiel 7

9 g Neopentylglykolcarbonat, 1 g Spiro-bis-dimethylencarbonat (erhalten gemäß Beispiel 6) und 0,01 g gemahlenes Kaliumcarbonat wurden auf 130 °C erhitzt. Unter plötzlicher Temperatursteigerung auf 150 °C trat Polymerisation zu einem unschmelzbaren und in Lösungsmitteln unlöslichen transparenten Polymerisat ein. Dieses zeichnet sich durch große Härte, hohe Kerbschlagzähigkeit und Elastizität aus.

Beispiel 8

Zu einer Suspension von 34 g (0,25 Mol) feingemahlenem Pentaerythrit, 200 ml getrocknetem Essigsäureethylester und 60,5 g (0,5 Mol) Dimethylanilin wurden 78,3 g (0,5 Mol) Chlorkohlensäurephenylester unter Rühren eintropfen gelassen und eine halbe Stunde auf 60 °C erwärmt. Nach Erkalten wurde zweimal mit Wasser ausgeschüttelt, über Natriumsulfat getrocknet und das Lösungsmittel unter vermindertem Druck entfernt. Der Rückstand (65 g) bestand im wesentlichen aus Pentaerythritbisphenylcarbonat. Er wurde auf 150 bis 200 °C bei 17 mbar erhitzt, bis die Abspaltung von Phenol (31 g) beendet war. 10 g des Rückstandes wurden in einer Sublimationsapparatur auf 230 °C bei 0,02 mbar erhitzt. Es wurden 1 g Kristalle vom Schmelzpunkt 220 bis 230 °C (Gasentwicklung) erhalten, bei denen es sich um Spiro-bis-dimethylencarbonat (s. Formel V) handelte.

Beispiel 9

134 g (1 Mol) Pentaerythrit, 710 g (6 Mol) Diethylcarbonat, 100 mg Thalliumcarbonat und 200 mg Bleinaphthenat wurden an einer 1 m-Füllkörperkolonne zum Rückfluß (125-130 °C) erhitzt, während über Kopf bei 78 bis 80 °C Ethanol abdestilliert wurde. Nach 18,5 Stunden waren 183 g Ethanol übergegangen. Der Rückstand (640 g) wurde mit 20 g handelsüblicher säureaktivierter Bleicherde 1 Stunde bei 100 °C gerührt. Die festen Bestandteile wurden mit einer Drucknutsche abgetrennt und das Filtrat unter einem Druck von 20 mbar bis zu einer Innentemperatur von 150 °C erhitzt und dabei vom überschüssigen Diethylcarbonat befreit (326 g). Durch Destillation des Rückstandes im Hochvakuum bei 190 bis 192 °C und 0,07 mbar wurden 255 g einer farblosen zähen Flüssigkeit erhalten. Diese wurde in Toluol gelöst und beim Abkühlen fielen 86 g Kristalle vom Schmelzpunkt 91 bis 92 °C aus. Diese Verbindung war das Pentaerythrit-monocarbonat-bis-ethylcarbonat (s. Formel (XIV)). Die eingeengte Mutterlauge wurde mit Methanol angerieben, wobei 153 g Kristalle vom Schmelzpunkt 55 bis 57 °C erhalten wurden. Diese Verbindung war das Pentaerythrit-tetrakis-ethylcarbonat (s. Formel XV).

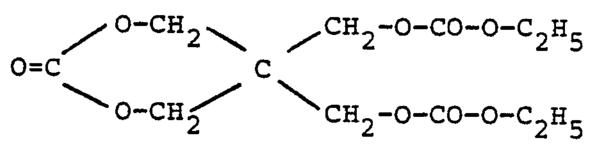

(XIV)

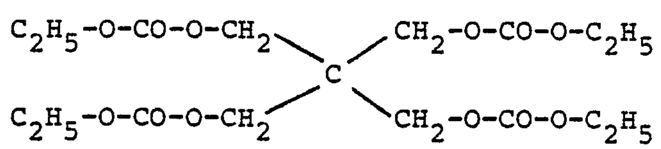

(XV)

Aus diesen beiden Derivaten des Pentaerythrits ließen sich durch Sublimation unter vermindertem Druck bicyclische Pentaerythritcarbonate wie folgt herstellen :

a) 2 g Pentaerythrit-monocarbonat-bis-ethylcarbonat (XIV) wurden mit 2 mg Dibutylzinndilaurat auf 220 °C erhitzt. Ca. 7 g Diethylcarbonat destillierten in die Vorlage. Der Rückstand war vernetzt. Er wurde in einer Sublimationsapparatur auf 220 bis 260 °C bei 0,03 mbar erhitzt. Aus dem Sublimat wurden 0,63 g Kristalle vom Schmelzpunkt 210 bis 230 °C (Gasentwicklung) erhalten. Es handelte sich um Spiro-bis-dimethylencarbonat (s. Formel V).

b) 4 g Pentaerythrit-tetrakis-ethylcarbonat (XV) wurden mit 2 mg Zinndilaurat auf 180 bis 220 °C erhitzt. 2 g Diethylcarbonat sammelten sich in der Vorlage. Der vernetzte Rückstand wurde in einer Sublimationsapparatur auf 220 bis 250 °C bei 0,02 mbar erhitzt. Das kristalline Sublimat wurde aus Essigsäureethylester umkristallisiert. Es wurden 0,8 g Kristalle vom Schmelzpunkt 220 bis 230 °C (Gasentwicklung) erhalten. Es handelte sich um Spiro-bis-dimethylencarbonat (s. Formel V).

Beispiel 10

20 g des gemäß Beispiel 6 nach Entfernung des Katalysators erhaltenen Rohproduktes wurden mit 10 mg Natriumhydroxid, gelöst in Methanol, vermischt und bei 20 mbar bis auf 200 °C erhitzt, wobei 1,3 g Diethylcarbonat übergingen. Bei 0,05 mbar wurde das Erhitzen auf 220 bis 240 °C fortgesetzt und ein bei 150 bis 180 °C überdestillierendes, vorwiegend kristallin erstarrendes Destillat (4,5 g) erhalten. Nach Umkristallisieren auf Essigsäureethylester lag der Schmelzpunkt bei 128 bis 130 °C. Es handelte sich um Oxetan-3,3-dimethylencarbonat (s. Formel VI).

Beispiel 11

125 g (0,5 Mol) Di-trimethylolpropan, 295 g (2,5 Mol) Diethylcarbonat und 0,5 g gemahlenes Kaliumcarbonat wurden an einer 1 m-Füllkörperkolonne am Rückfluß erhitzt, währenddessen über Kopf bei 78 °C Ethanol abdestillierte. Nach 2,5 Stunden waren 90 g Ethanol abgetrennt. Die Sumpftemperatur überstieg dabei nicht 130 °C. Das Reaktionsprodukt wurde in 600 ml Methylenchlorid aufgenommen und die so erhaltene Lösung durch ein mit 200 g eines Ionenaustauschers, bestehend aus einem sulfonierten, vernetzten Polystyrol, beschicktes Rohr gegeben. Nach Einengen der Lösung bei Temperaturen bis zu 90 °C und 15 mbar wurden 211 g Rückstand erhalten.

86 g dieses Rückstandes wurden nach Zugabe von 100 mg Zinndilaurat unter Rühren während einer Stunde von 160 auf 230 °C bei 0,01 mbar erhitzt. Das kurz danach vorliegende stark vernetzte Polycarbonat wurde auf Temperaturen von 260 bis 290 °C bei 0,01 mbar gebracht, wobei zwischendurch wieder Verflüssigung eintrat, während 60 g zähes, in der Kälte kristallisierendes Destillat bei 250 bis 265 °C übergingen. In einer dem Auffanggefäß für das Destillat nachgeschalteten, mit Trockeneis beschickten Kühlfalle wurden 24 g Diethylcarbonat und in einer weiteren, mit flüssigem Stickstoff gekühlten Kühlfalle 1 g Kohlendioxid aufgefangen. Das erhaltene Destillat wurde aus 250 ml Toluol umkristallisiert. Es handelte sich um Di-trimethylolpropan-dicarbonat der Formel (III), das in einer Ausbeute von 49 g entsprechend 79 % der Theorie erhalten wurde. Der Schmelzpunkt betrug nach der Umkristallisation 102 bis 103 °C.

Elementaranalyse : $C_{14}H_{22}O_7$ (302,3)
C ber. : 55,62 gef. : 55,33
H ber. : 7,34 gef. : 7,51
O ber. : 37,05 gef. : 37,30

Beispiel 12

9 g Neopentylglykolcarbonat, 1 g Di-trimethylolpropandicarbonat (erhalten gemäß Beispiel 11) und 0,1 g Kaliumcarbonat wurden auf 130 °C erhitzt. Unter Temperaturerhöhung trat Polymerisation zu einem unschmelzbaren, im Lösungsmittel unlöslichen, transparenten Polymerisat von großer Zähe, Härte und Elastizität ein.

Beispiel 13

Es wurde gearbeitet wie in Beispiel 11 bis einschl. der Behandlung mit dem Ionenaustauscher und dem Einengen der Lösung. 90 g des so erhaltenen Rückstandes wurden ohne Zusatz eines Katalysators 1,5 Stunden auf 180 bis 220 °C erhitzt. Währenddessen sammelten sich in der mit Trockeneis beschickten Vorlage 26 g Diethylcarbonat. Das eigentliche Reaktionsprodukt wurde bei 250 bis 295 °C Innentemperatur und 260 bis 280 °C Destillationstemperatur bei 0,01 mbar abdestilliert. Es wurden 62 g einer kristallisierenden Masse erhalten, die aus Toluol umkristallisiert wurde, worauf 48 g Kristalle vom Schmelzpunkt 100 bis 102 °C anfielen. Die Ausbeute betrug 74 % der Theorie. Es handelte sich um die gleiche Verbindung wie die in Beispiel 11 erhaltene.

**Ansprüche**

1. Cyclische Kohlensäurederivate der Formel

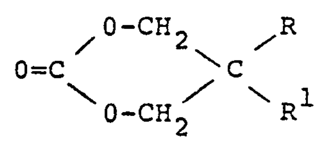

in der R entweder $—CH_2OH$ bedeutet, wobei dann $R^1$ für einen Ethylrest steht, oder

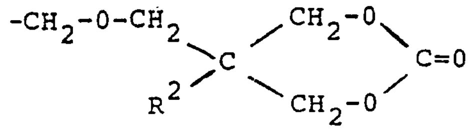

bedeutet, wobei dann $R^1$ und $R^2$ unabhängig voneinander für Methyl oder Ethyl stehen, oder

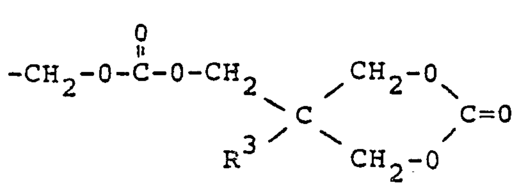

bedeutet, wobei dann $R^1$ und $R^3$ jeweils für einen Alkylrest mit 1 bis 4 C-Atomen stehen, oder in der R und $R^1$ zusammen

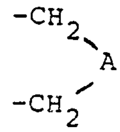

bedeuten, wobei A für die Gruppe

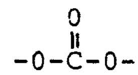

oder für Sauerstoff steht.

2. 1,3-Dioxan-5-hydroxymethyl-5-ethyl-2-on
3. 5,5'-[Oxybis (methylen)] bis [5-ethyl-1,3-dioxan-2-on]
4. 5,5'-[Carbonylbis(oxymethylen] bis [5-ethyl-1,3-dioxan-2-on]
5. 2,4,7,9-Tetraoxa-spiro [5,5] undecandion (3,8)
6. 2,4,7-Trioxa-spiro [3,5] nonanon (3)
7. Verfahren zur Herstellung von cyclischen Kohlensäurederivaten der Formel

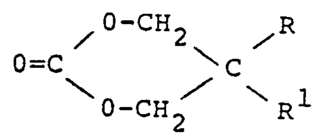

in der R entweder $—CH_2OH$ bedeutet, wobei dann $R^1$ für einen Alkylrest mit 1 bis 4 C-Atomen steht (Fall 1), oder

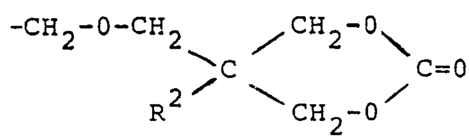

bedeutet, wobei dann $R^1$ und $R^2$ unabhängig voneinander für Methyl oder Ethyl stehen (Fall 2), oder

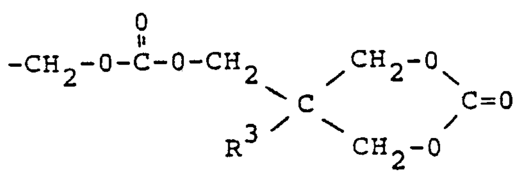

bedeutet, wobei dann $R^1$ und $R^3$ jeweils für einen Alkylrest mit 1 bis 4 C-Atomen stehen (Fall 3), oder in der R und $R^1$ zusammen

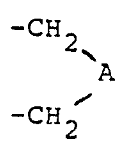

bedeuten, wobei A für die Gruppe

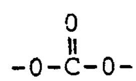

oder für Sauerstoff steht (Fall 4), dadurch gekennzeichnet, daß man ein Polyol der Formel

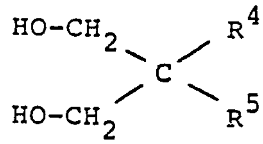

in der $R^4$ entweder $—CH_2OH$ bedeutet, wobei dann $R^5$ für einen Alkylrest mit 1 bis 4 C-Atomen oder für $—CH_2OH$ steht oder

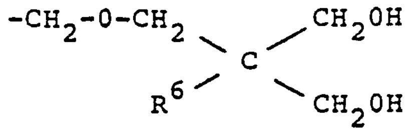

bedeutet, wobei dann $R^5$ und $R^6$ unabhängig voneinander für Methyl oder Ethyl stehen, mit einem Kohlensäurederivat aus der Gruppe, die Phosgen, Chlorkohlensäureester, Kohlensäuredialkylester und Kohlensäurediarylester umfaßt, zu einem löslichen Polycarbonat umsetzt, in denjenigen Fällen, in denen $R^4$ für $—CH_2OH$ und $R^5$ für einen Alkylrest mit 1 bis 4 C-Atomen oder $R^4$ für den Rest

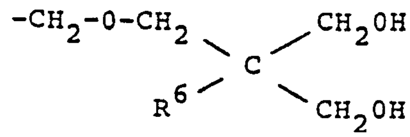

steht, den bei der Umsetzung zu einem löslichen Polycarbonat verwendeten Katalysator zwingend, in denjenigen Fällen, in denen $R^4$ für $—CH_2OH$ und $R^5$ für $—CH_2OH$ steht, diesen Katalysator gegebenenfalls entfernt, danach in den Fällen 2 und 4 das lösliche Polycarbonat in ein vernetztes, unlösliches Polycarbonat überführt, und zwar im Fall 2 bei Temperaturen von 150 bis 240 °C und Drucken von 0,001 bis 10 mbar und im Fall 4 bei Temperaturen von 100 bis 250 °C und Drucken von 1 bis 140 mbar, das dann vorliegende lösliche oder unlösliche Polycarbonat depolymerisiert, und zwar in den Fällen 1 und 3 bei Temperaturen von 150 bis 240 °C und Drucken von 0,001 bis 25 mbar, im Fall 2 bei Temperaturen von 240 bis 320 °C und Drucken von 0,001 bis 2 mbar und im Fall 4 bei Temperaturen von 200 bis 300 °C und Drucken von 0,001 bis 1 mbar, und die sich dabei bildenden cyclischen Kohlensäurederivate abtrennt.

8. Verfahren nach Anspruch 7, dadurch gekennzeichnet, daß man cyclische Kohlensäurederivate, bei denen R für $—CH_2OH$ oder $R^1$ für $C_1$- bis $C_4$-Alkyl steht, herstellt, indem man aus einem Trimethylolalkan der Formel

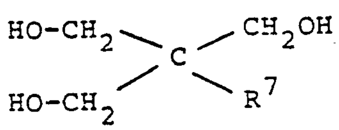

$R^7 = C_1$- bis $C_4$-Alkyl

und einer äquimolaren Menge eines Kohlensäurederivates aus der Gruppe, die Phosgen, Chlorkohlen säureester, Kohlensäuredialkylester und Kohlensäurediarylester umfaßt, unter Zusatz von Natrium- oder Kaliumhydroxid, -carbonat oder -phenolat als Katalysator ein lösliches Polycarbonat herstellt, die verwendeten Katalysatoren, deren Folgeprodukte und/oder sonstige Fremdstoffe entfernt, das so vorbehandelte lösliche Polycarbonat auf Temperaturen von 150 bis 240 °C erhitzt und die sich dabei bildenden cyclischen Kohlensäurederivate abtrennt.

9. Verfahren nach Anspruch 7, dadurch gekennzeichnet, daß man cyclische Kohlensäurederivate, bei denen R und $R^1$ zusammen für

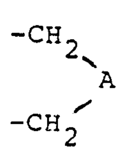

steht, wobei A für die Gruppe

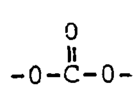

oder Sauerstoff steht, herstellt, indem man

a) Pentaerythrit mit einem Kohlensäurederivat aus der Gruppe, die Phosgen, Chlorkohlensäureester, Kohlensäuredialkylester und Kohlensäurediarylester umfaßt, gegebenenfalls in Gegenwart basisch wirkender Verbindungen, zu einem löslichen Vorprodukt umsetzt,

b) danach gegebenenfalls die basisch wirkenden Verbindungen entfernt,

c) aus dem löslichen Vorprodukt durch Erhitzen auf 100 bis 250 °C bei Drucken im Bereich von 1 bis 140 mbar ein vernetztes Polycarbonat herstellt,

d) das vernetzte Polycarbonat, gegebenenfalls in Gegenwart von Katalysatoren, bei 200 bis 300 °C und Drucken im Bereich von 0,001 bis 1 mbar depolymerisiert und

e) die im Schritt d) absublimierenden oder abdestillierenden bicyclischen Kohlensäurederivate auffängt und gegebenenfalls trennt.

10. Verfahren nach Anspruch 7, dadurch gekennzeichnet, daß man cyclische Kohlensäurederivate, bei denen R für

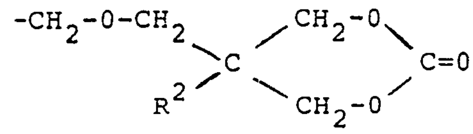

steht und $R^1$ und $R^2$ unabhängig voneinander für Methyl oder Ethyl stehen, herstellt, indem man Ditrimethylolalkane der Formel

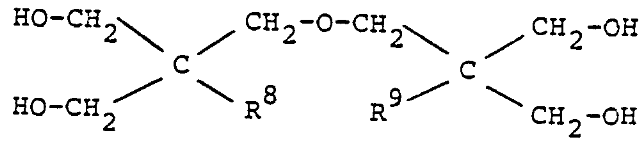

in der $R^8$ und $R^9$ gleich oder verschieden sein können und für Methyl oder Ethyl stehen,

a) mit einem Kohlensäurederivat aus der Gruppe, die Phosgen, Chlorkohlensäureester, Kohlensäuredialkylester und Kohlensäurediarylester umfaßt, zu einem löslichen Vorprodukt umsetzt,

b) falls im Schritt a) in Gegenwart basisch wirkender Verbindung gearbeitet wurde, diese basisch wirkenden Verbindungen entfernt,

c) in Abwesenheit von Katalysatoren oder in Anwesenheit schwach wirksamer Umesterungskatalysatoren aus der Gruppe der organischen Verbindungen des 2- und 4-wertigen Zinns und des 4-wertigen Titans durch Erhitzen auf Temperaturen im Bereich 150 bis 240 °C und bei Drucken im Bereich von 0,001 bis 10 mbar aus dem löslichen Vorprodukt ein vernetztes Polycarbonat herstellt und

d) das vernetzte Polycarbonat bei Temperaturen von 240 bis 320 °C und Drucken im Bereich von 0,001 bis 2 mbar depolymerisiert und das übergehende bicyclische Ethercarbonat in einer gekühlten Vorlage auffängt.

11. Verfahren nach Anspruch 7, dadurch gekennzeichnet, daß man cyclische Kohlensäureester, bei denen R für

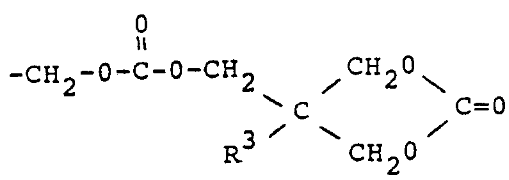

steht und $R^1$ und $R^3$ jeweils für $C_1$- bis $C_4$-Alkyl stehen, herstellt, indem man entsprechend Anspruch 8 verfährt und anschließend die erhaltenen Produkte mit Phosgen in Gegenwart basischer Verbindungen umsetzt.

12. Verwendung von cyclischen Kohlensäureestern der Formel

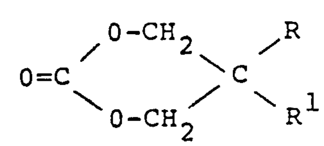

(I)

in der R entweder $—CH_2OH$ bedeutet, wobei dann $R^1$ für einen Alkylrest mit 1 bis 4 C-Atomen steht, oder

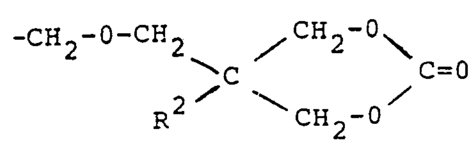

bedeutet, wobei dann $R^1$ und $R^2$ unabhängig voneinander für Methyl oder Ethyl stehen, oder

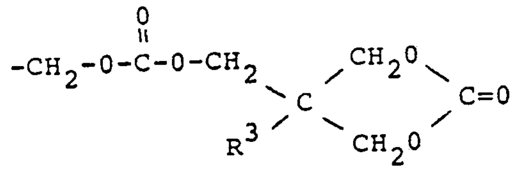

bedeutet, wobei dann $R^1$ und $R^3$ jeweils für einen Alkylrest mit 1 bis 4 C-Atomen stehen, oder in der R und $R^1$ zusammen

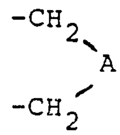

bedeuten, wobei A für die Gruppe

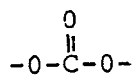

oder für Sauerstoff steht, als Copolymerisationskomponenten zur Herstellung von Polycarbonaten.

**Claims**

1. Cyclic carbonic acid derivatives of the formula

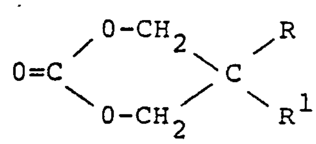

in which R either denotes $-CH_2OH$, $R^1$ then representing an ethyl radical, or denotes

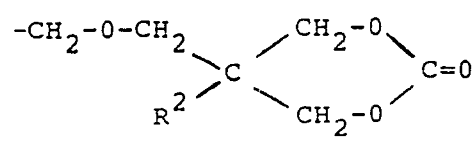

$R^1$ and $R^2$ then independently of one another representing methyl or ethyl, or denotes

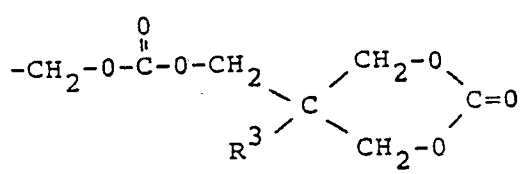

$R^1$ an $R^3$ then each representing an alkyl radical having 1 to 4 C atoms, or in which R and $R^1$ together denote

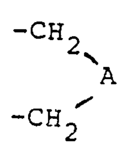

wherein A represents the group

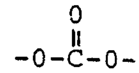

or oxygen.

2. 1,3-Dioxane-5-hydroxymethyl-5-ethyl-2-one
3. 5,5′-[Oxybis(methylene)] bis [5-ethyl-1,3-dioxan-2-one]
4. 5,5′-[Carbonylbis(oxymethylene)] bis [5-ethyl-1,3-dioxan-2-one]
5. 2,4,7,9-Tetraoxa-spiro [5,5] undecane-3,8-dione
6. 2,4,7-Trioxa-spiro [3,5] nonan-3-one
7. Process for the preparation of cyclic carbonic acid derivatives of the formula

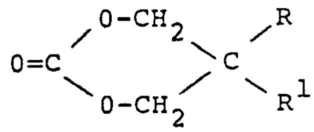

in which R either denotes $—CH_2OH$, $R^1$ then representing an alkyl radical with 1 to 4 C atoms (case 1), or denotes

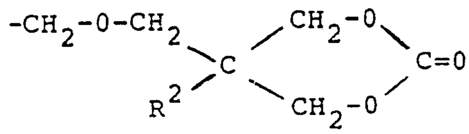

$R^1$ and $R^2$ then independently of one another representing methyl or ethyl (case 2), or denotes

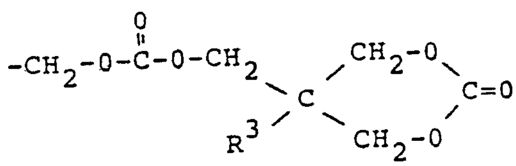

$R^1$ and $R^3$ then each representing an alkyl radical with 1 to 4 C atoms (case 3), or in which R and $R^1$ together denote

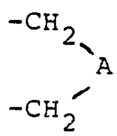

wherein A represents the group

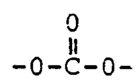

or oxygen (case 4), characterised in that a polyol of the formula

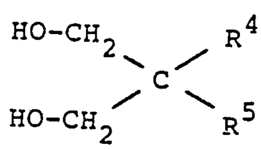

in which $R^4$ either denotes $—CH_2OH$, $R^5$ then representing an alkyl radical with 1 to 4 C atoms or $—CH_2OH$, or denotes

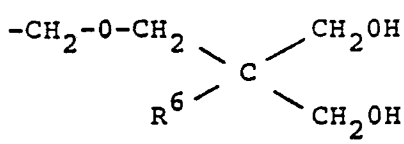

$R^5$ and $R^6$ then independently of one another representing methyl or ethyl, is reacted with a carbonic acid derivative from the group comprising phosgene, chlorocarbonates, dialkyl carbonates and diaryl carbonates, to give a soluble polycarbonate, in those cases in which $R^4$ represents $-CH_2OH$ and $R^5$ represents an alkyl radical with 1 to 4 C atoms or $R^4$ represents the radical

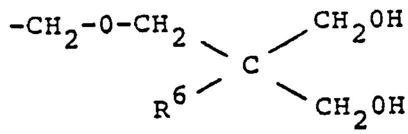

the catalyst used in the reaction to give a soluble polycarbonate must be removed, and in those cases in which $R^4$ represents $-CH_2OH$ and $R^5$ represents $-CH_2OH$, this catalyst is optionally removed, then in cases 2 and 4 the soluble polycarbonate is converted into a crosslinked insoluble polycarbonate, in case 2 at temperatures of 150 to 240 °C and under pressures of 0.001 to 10 mbars and in case 4 at temperatures of 100 to 250 °C and under pressures of 1 to 140 mbars, the soluble or insoluble polycarbonate then present is depolymerised, in cases 1 and 3 at temperatures of 150 to 240 °C and under pressures of 0.001 to 25 mbars, in case 2 at temperatures of 240 to 320 °C and under pressures of 0.001 to 2 mbars and in case 4 at temperatures of 200 to 300 °C and under pressures of 0.001 to 1 mbar, and the cyclic carbonic acid derivatives formed in the process are separated off.

8. Process according to Claim 7, characterized in that cyclic carbonic acid derivatives in which R represents $-CH_2OH$ or $R^1$ represents $C_1$- to $C_4$-alkyl, are prepared by preparing a soluble polycarbonate from a trimethylolalkane of the formula

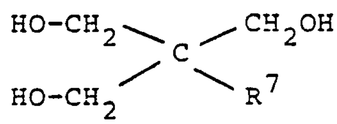

$R^7 = C_1$- to $C_4$-alkyl

and an equimolar quantity of a carbonic acid derivative from the group comprising phosgene, chlorocarbonates, dialkyl carbonates and diaryl carbonates, with the addition of sodium hydroxide, carbonate or phenolate or potassium hydroxide, carbonate or phenolate as the catalyst, removing the catalysts used, the by-products thereof and/or other extraneous compounds, heating the soluble polycarbonate thus pre-treated to temperatures of 150 to 240 °C and separating off the cyclic carbonic acid derivatives formed in the process.

9. Process according to Claim 7, characterised in that cyclic carbonic acid derivatives in which R and $R^1$ together represent

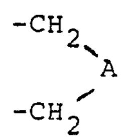

wherein A represents the group

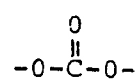

or oxygen, are prepared by

a) reacting pentaerythritol with a carbonic acid derivative from the group comprising phosgene, chlorocarbonates, dialkyl carbonates and diaryl carbonates, optionally in the presence of basic compounds, to give a soluble precursor,

b) if appropriate thereafter removing the basic compounds,

c) preparing a crosslinked polycarbonate from the soluble precursor by heating the latter to 100 to 250 °C under pressures in the range of 1 to 140 mbars,

d) depolymerising the crosslinked polycarbonate, optionally in the presence of catalysts, at 200 to 300 °C and under pressures in the range of 0.001 to 1 mbar and

e) collecting and, if appropriate, separating the bicyclic carbonic acid derivatives which sublime or distil off in step d).

10. Process according to Claim 7, characterised in that cyclic carbonic acid derivatives in which R represents

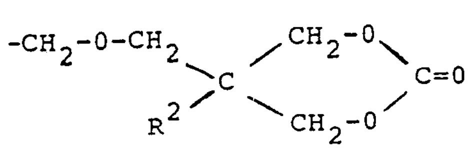

and $R^1$ and $R^2$ independently of one another represent methyl or ethyl, are prepared by reacting di-trimethylolalkanes of the formula

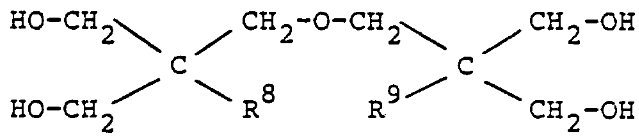

in which $R^8$ and $R^9$ can be identical or different and represent methyl or ethyl,

a) with a carbonic acid derivative from the group comprising phosgene, chlorocarbonates, dialkyl carbonates and diaryl carbonates, to give a soluble precursor,

b) if, in step a), the reaction was carried out in the presence of a basic compound, removing these basic compounds,

c) preparing a crosslinked polycarbonate from the soluble precursor, in the absence of catalysts or in the presence of weak transesterification catalysts from the group of organic compounds of divalent and tetravalent tin and of tetravalent titanium, by heating to temperatures in the range of 150 to 240 °C and under pressures in the range of 0.001 to 10 mbars, and

d) depolymerising the crosslinked polycarbonate at temperatures of 240 to 320 °C and under pressures in the range of 0.001 to 2 mbars and collecting the bicyclic ether carbonate which passes over, in a cooled receiver.

11. Process according to Claim 7, characterised in that cyclic carbonates in which R represents

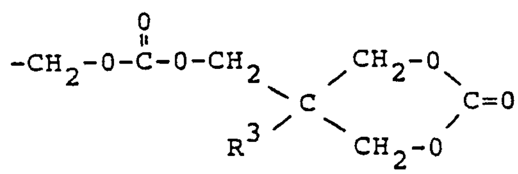

and $R^1$ and $R^3$ each represent $C_1$- to $C_4$-alkyl, are prepared by proceeding according to Claim 8 and then reacting the resulting products with phosgene in the presence of basic compounds.

12. Use of cyclic carbonates of the formula

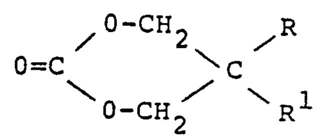

(I)

in which R either denotes $-CH_2OH$, $R^1$ then representing an alkyl radical with 1 to 4 C atoms, or denotes

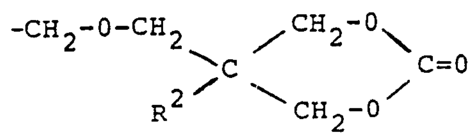

$R^1$ and $R^2$ then independently of one another representing methyl or ethyl, or denotes

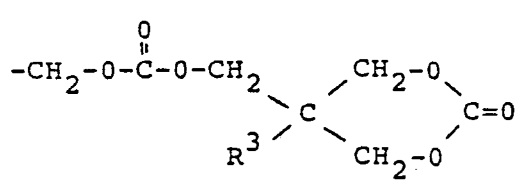

$R^1$ and $R^3$ then each representing an alkyl radical with 1 to 4 C atoms, or in which R and $R^1$ together denote

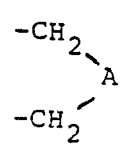

wherein A represents the group

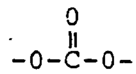

or oxygen, as copolymerisation components for the preparation of polycarbonates.

**Revendications**

1. Dérivés cycliques de l'acide carbonique de formule

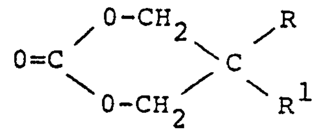

dans laquelle ou bien R représente —$CH_2OH$ et $R^1$ représente alors un groupe éthyle, ou bien R représente

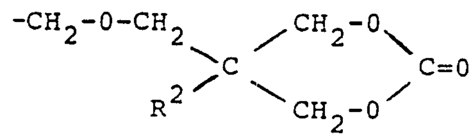

et $R^1$ et $R^2$ représentent alors, indépendamment l'un de l'autre, des groupes méthyle ou éthyle, ou bien R représente

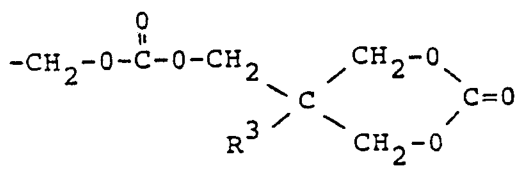

et $R^1$ et $R^3$ représentent alors chacun un groupe alkyle en $C_1$-$C_4$, ou bien R et $R^1$ forment ensemble le groupe

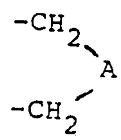

dans lequel A représente le groupe

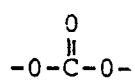

ou l'oxygène.

2. La 1,3-dioxanne-5-hydroxyméthyl-5-éthyl-2-one.
3. La 5,5'-[oxy-bis-(méthylène)] bis [5-éthyl-1,3-dioxanne-2-one].
4. La 5,5'-[carbonyl-bis(oxyméthylène)] bis [5-éthyl-1,3-dioxanne-2-one].
5. La 2,4,7,9-tétraoxa-spiro [5,5] undécanedione-(3,8).
6. La 2,4,7-trioxa-spiro [3,5] nonanone-(3).
7. Procédé de préparation de dérivés cycliques de l'acide carbonique de formule

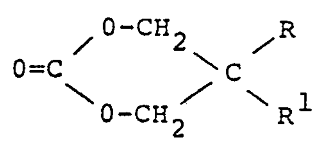

dans laquelle ou bien R représente —$CH_2OH$, et $R^1$ représente alors un groupe alkyle en $C_1$-$C_4$ (cas 1) ; ou bien R représente

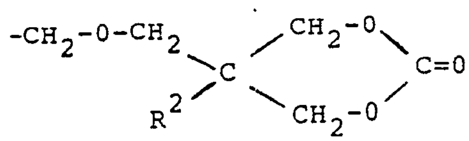

et $R^1$ et $R^2$ représentent alors, indépendamment l'un de l'autre, des groupes méthyle ou éthyle (cas 2), ou bien R représente

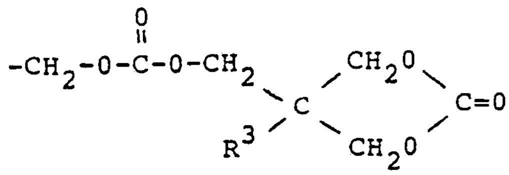

et $R^1$ et $R^3$ représentent alors chacun un groupe alkyle en $C_1$-$C_4$ (cas 3), ou bien R et $R^1$ forment ensemble le groupe

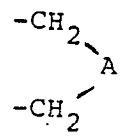

dans lequel A représente le groupe

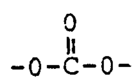

ou l'oxygène (cas 4), caractérisé en ce que l'on fait réagir un polyol de formule

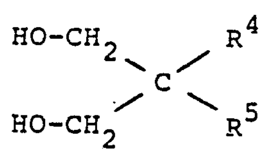

dans laquelle ou bien $R^4$ représente —$CH_2OH$, et $R^5$ représente alors un groupe alkyle en $C_1$-$C_4$ ou un groupe —$CH_2OH$, ou bien $R^4$ représente

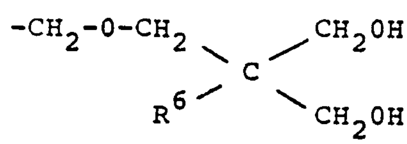

et $R^5$ et $R^6$ représentent alors, indépendamment l'un de l'autre, des groupes méthyle ou éthyle, avec un dérivé de l'acide carbonique du groupe consistant en le phosgène, les esters chlorocarboniques, le carbonate de dialkyle et le carbonate de diaryle, formant ainsi un polycarbonate soluble, et dans le cas où $R^4$ représente —$CH_2OH$ et $R^5$ un groupe alkyle en $C_1$-$C_4$ ou bien $R^4$ représente le groupe

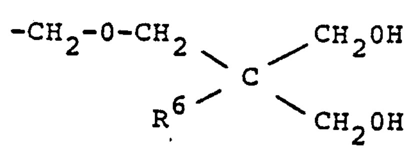

on élimine obligatoirement le catalyseur utilisé dans la réaction conduisant à un polycarbonate soluble, et, dans le cas où $R^4$ représente —$CH_2OH$ et $R^5$ représente —$CH_2$—OH, on élimine éventuellement ce

catalyseur puis, dans les cas 2 et 4, on convertit le polycarbonate soluble en un polycarbonate réticulé insoluble, à savoir dans le cas 2 à des températures de 150 à 240 °C et des pressions de 0,001 à 10 mbar et dans le cas 4 à des températures de 100 à 250 °C et des pressions de 1 à 140 mbar, puis on dépolymérise le polycarbonate soluble ou insoluble obtenu, à savoir dans les cas 1 et 3 à des températures de 150 à 240 °C et des pressions de 0,001 à 25 mbar, dans le cas 2 à des températures de 240 à 320 °C et des pressions de 0,001 à 2 mbar et dans le cas 4 à des températures de 200 à 300 °C et des pressions de 0,001 à 1 mbar, et on sépare les dérivés cycliques de l'acide carbonique ainsi formés.

8. Procédé selon la revendication 7, caractérisé en ce que l'on prépare les dérivés cycliques de l'acide carbonique dans lesquels R représente $-CH_2OH$ ou bien $R^1$ représente un groupe alkyle en $C_1$-$C_4$, en préparant à partir d'un triméthylolalcane de formule

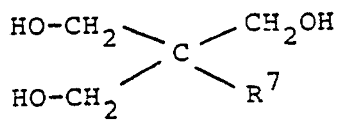

dans laquelle $R^7$ représente un groupe alkyle en $C_1$-$C_4$ et d'une quantité équimoléculaire d'un dérivé de l'acide carbonique pris dans le groupe consistant en le phosgène, les esters chlorocarboniques, les carbonates de dialkyle et les carbonates de diaryle, avec adjonction d'hydroxyde, de carbonate ou de phénate de sodium ou de potassium en tant que catalyseur, un polycarbonate soluble, on élimine les catalyseurs utilisés, leurs produits de transformation et/ou autres substances étrangères éventuelles, on chauffe le polycarbonate soluble qui a subi ce traitement à des températures de 150 à 240 °C et on sépare les dérivés cycliques de l'acide carbonique ainsi formés.

9. Procédé selon la revendication 7, caractérisé en ce que l'on prépare des dérivés cycliques de l'acide carbonique dans lesquels R et $R^1$ forment ensemble le groupe

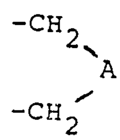

dans lequel A représente le groupe

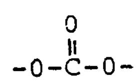

ou l'oxygène,

a) en faisant réagir le pentaérythritol avec un dérivé de l'acide carbonique pris dans le groupe consistant en le phosgène, les esters chlorocarboniques, les carbonates de dialkyle et les carbonates de diaryle, éventuellement en présence de composés à réaction basique, avec formation d'un produit intermédiaire soluble,

b) en éliminant ensuite éventuellement les composés à réaction basique,

c) en préparant à partir du produit intermédiaire soluble, par chauffage à une température de 100 à 250 °C sous des pressions dans l'intervalle de 1 à 140 mbar un polycarbonate réticulé,

d) en dépolymérisant le polycarbonate réticulé, éventuellement en présence de catalyseurs, à des températures de 200 à 300 °C et des pressions dans l'intervalle de 0,001 à 1 mbar, et

e) en recueillant les dérivés cycliques de l'acide carbonique qui subliment ou distillent dans le stade opératoire d) et le cas échéant en les séparant.

10. Procédé selon la revendication 7, caractérisé en ce que l'on prépare les dérivés cycliques de l'acide carbonique dans lesquels R représente

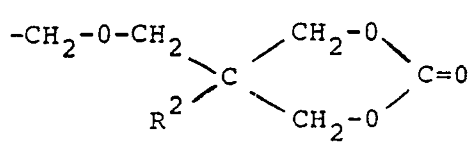

et $R^1$ et $R^2$ représentent chacun, indépendamment l'un de l'autre, un groupe méthyle ou éthyle, en faisant réagir des di-triméthylolalcanes de formule

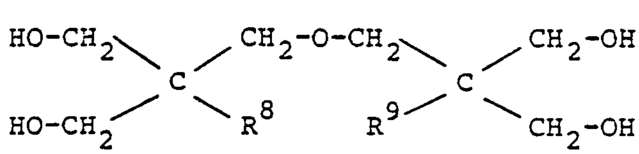

dans laquelle $R^8$ et $R^9$, identiques ou différents, représentent des groupes méthyle ou éthyle,

a) avec un dérivé de l'acide carbonique pris dans le groupe consistant en le phosgène, les esters chlorocarboniques, les carbonates de dialkyle et les carbonates de diaryle, avec formation d'un produit intermédiaire soluble,

b) lorsqu'on a travaillé au stade opératoire a) en présence d'un composé à réaction basique, en éliminant ce composé à réaction basique,

c) en préparant à partir du produit intermédiaire soluble un polycarbonate réticulé en l'absence de catalyseurs ou en présence de catalyseurs de transestérification peu actifs du groupe des composés organiques de l'étain trivalent et tétravalent et du titane tétravalent par chauffage à des températures dans l'intervalle de 150 à 240 °C sous des pressions dans l'intervalle de 0,001 à 10 mbar, et

d) en dépolymérisant le polycarbonate réticulé à des températures de 240 à 320 °C et des pressions dans l'intervalle de 0,001 à 2 mbar et en recueillant l'éther-carbonate bicyclique qui passe dans un récepteur refroidi.

11. Procédé selon la revendication 7, caractérisé en ce que l'on prépare les esters cycliques de l'acide carbonique dans lesquels R représente

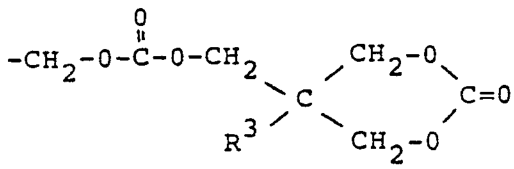

et $R^1$ et $R^3$ représentent chacun un groupe alkyle en $C_1$-$C_4$, en opérant comme décrit dans la revendication 8 puis en faisant réagir les produits obtenus avec du phosgène en présence de composés basiques.

12. Utilisation des esters cycliques de l'acide carbonique de formule

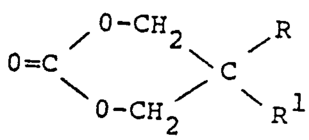

(I)

dans laquelle ou bien R représente —$CH_2OH$, et $R^1$ représente alors un groupe alkyle en $C_1$-$C_4$, ou bien R représente

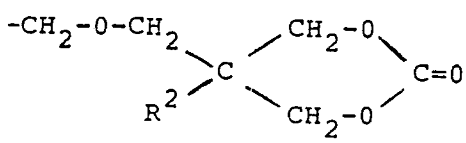

et $R^1$ et $R^2$ représentent alors, indépendamment l'un de l'autre, des groupes méthyle ou éthyle, ou bien R représente

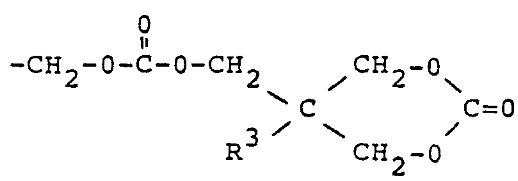

et $R^1$ et $R^3$ représentent alors chacun un groupe alkyle en $C_1$-$C_4$, ou bien R et $R^1$ forment ensemble le groupe

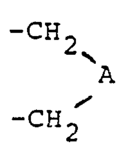

dans lequel A représente le groupe

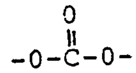

ou l'oxygène, en tant que composants de copolymérisation dans la préparation de polycarbonates.